# EUROPEAN PATENT APPLICATION

(11) **EP 1 712 180 A1**
(43) Date of publication of application: **18.10.2006**
(21) Application number: 05014256.1
(22) Date of filing: 30.06.2005
(51) Int. Cl.: A61B 5/151

(54) **Blood collecting kit**

(30) Priority: 11.04.2005 JP 2005112990
(71) Applicant: Enomoto Co., Ltd., Kita-tsuru-gun, Yamanashi-ken (JP)
(72) Inventor: Hosoda, Mitsuji, Yamanashi, 409-0114 (JP); Ohkanda, Kouki, Yamanashi, 409-0115 (JP); Osada, Yoshimichi, Yamanashi, 409-0112 (JP); Enomoto, Akihiro, Yamanashi, 401-0502 (JP)
(74) Representative: Luderschmidt, Schüler & Partner

(57) **Abstract**

A blood collecting device according to the present invention is a blood collecting device, wherein a columnar liquid absorber, which contains a blood coagulation preventing solution, has a slit, and temporarily absorbs a predetermined amount of blood without coagulation is disposed at a tip end, and a pressing body which presses a rear surface of the liquid absorber to separate the liquid absorber is provided.

## Description

### BACKGROUND OF THE INVENTION

### Field of the Invention

The present invention relates to a blood collecting kit including a blood collecting device and a blood collecting bottle. The present invention relates to a blood collecting kit suitable for collecting a specified amount of blood, saliva, pus, body fluid, or urine (hereinafter, called "blood or the like") for oneself at a hospital, one's own house or the like.

### Description of the Related Art

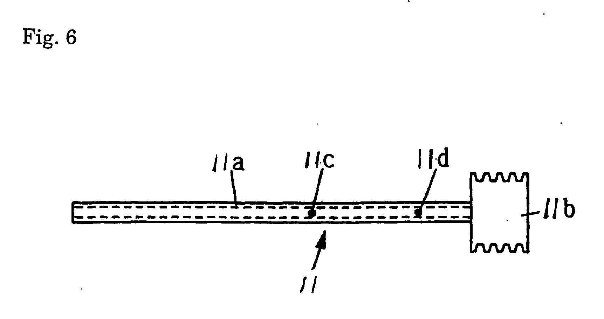

FIG. 6 is a schematic view of a conventional disposable blood collecting pipette. A conventional disposable pipette 11 is made by connecting a dropper tube 11a and a cap 11b of a bellows structure, and the dropper tube 11a and the cap 11b are made of a transparent or semitransparent material having flexibility. Marks 11c and 11d are put on the dropper tube 11a. The mark 11c shows a lower limit of an amount of blood or the like to be collected, and the mark 11d shows an upper limit of the amount of blood or the like to be collected. A tip end of the dropper 11a is opened, but has the same outer diameter and inner diameter as the other portions.

However, it is difficult to set the amount of collected blood or the like between the mark 11c and the mark 11d, and unless suction and discharge of the blood or the like are repeated by pressing and releasing the cap many times, a predetermined amount of blood or the like cannot be collected.

Air bubbles are easily included in the blood or the like in the pipette, the collected amount becomes small, and inaccurate. While a tip end of the dropper tube 5 is pinched with fingers of one hand, the tip end of the dropper tube is put into the blood or the like, and the blood or the like is collected into the dropper tube by capillarity. However, since the cap is pressed and released with a thumb, the operation is difficult for an unskilled person, and air is easily sucked by an erroneous operation. It is difficult to remove the included air with the conventional pipette. As a result, suction and discharge of the blood or the like have to be repeated many times. With the blood or the like in which air bubbles are included, sufficient collection amount cannot be obtained, and accurate inspection cannot be made.

### SUMMARY OF THE INVENTION

An object of the present invention is to provide a blood collecting kit including a relatively low price blood collecting device and a blood collecting bottle which can easily and accurately collect a predetermined amount of blood or the like without inclusion of air bubbles.

The above described object is achieved by a blood collecting device according to the present invention described in claim 1, namely, a blood collecting device wherein a columnar liquid absorber, which contains a blood coagulation preventing solution, has a slit, and temporarily absorbs a predetermined amount of blood without coagulation, is disposed at a tip end, and a pressing body which presses a rear surface of the liquid absorber to separate the liquid absorber is provided.

In a preferred embodiment according to the present invention, as described in claim 2, a position of the center of gravity is designed so that a tip end does not contact a surface of a table when the blood collecting device is placed on the table with its center axis nearly horizontal, and a projection which prevents the blood collecting device from rolling on the table is provided at a central part.

In another preferred embodiment of the blood collecting device according to the present invention, as described in claim 3, the blood collecting device has releasable lock means which stops movement of the pressing body to stop the pressing body from pressing the absorber, and releases the stoppage to make movement of the pressing body possible.

Further, in a blood collecting bottle according to the present invention, as described in claim 4, when the blood collecting device according to claim 3 is pushed and fitted therein, the blood collecting device abuts on an inner wall and releasable lock means of the blood collecting device is released, and when the pressing body of the blood collecting device is pressed, a liquid absorber is separated from the tip end of the blood collecting device, and the liquid absorber is immersed in a contained drug solution, and the blood temporarily absorbed in the liquid absorber elutes into the drug solution.

The blood collecting device according to the present invention is mounted with the liquid absorber at a tip end, and therefore, the effect of being capable of collecting a predetermined amount (for example, 1 µl, 10 µl, 20 µl, 50 µl, 100 µl) of blood or the like accurately and easily is provided.

Since the blood collecting device according to the present invention is provided with the pressing body which presses the rear surface of the liquid absorber and separates the liquid absorber, the liquid absorber which temporarily absorbs a predetermined amount of blood or the like can be separated and put into the blood collecting bottle according to the present invention, and the effect of being capable of immersing and storing the blood or the like into the drug solution in the blood collecting bottle and treating the blood or the like is provided. Especially because the liquid absorber has the slit, the effect that the blood or the like temporarily absorbed in the liquid absorber all elutes into the drug solution in a short time when the liquid absorber is immersed in the drug solution is provided.

Since the position of the center of the gravity is designed so that the tip end does not contact the surface of the table in the blood collecting device according to the present invention, the effect that the tip end hardly contacts the table surface and the like and is hygienic is provided.

Since the blood collecting device according to the present invention is provided with the projection at the center, the effect that the blood collecting device is difficult to roll and difficult to fall is provided.

Since the blood collecting device according to the present invention has the lock means which temporarily stops the movement of the pressing body, the effect that the accident of pressing and separating the liquid absorber by mistake when temporarily absorbing blood or the like does not happen is provided.

### BRIEF DESCRIPTION OF THE DRAWINGS

FIGS. 1A, 1B and 1C are schematic views of a blood collecting device of an embodiment according to the present invention;
FIG. 2 is a schematic view of the blood collecting device of the embodiment according to the present invention;
FIG. 3 is a schematic view of the blood collecting device and a blood collecting bottle of the embodiment according to the present invention;
FIG. 4 is a schematic view of the blood collecting device and the blood collecting bottle of the embodiment according to the present invention;
FIGS. 5A and 5B are perspective views of a liquid absorber (filter) used in the blood collecting device of the embodiment according to the present invention; and
FIG. 6 is a schematic view of a conventional disposable blood collecting pipette.

### [Description of Symbols]

- 5: dropper tube
- 11: disposable pipette
- 11a: dropper tube
- 11b: cap
- 11c: mark
- 11b: mark
- 21: body
- 21a: tip end portion
- 21b: small projection
- 21c: rear end portion
- 22: pressing shaft portion
- 22a: pressing portion
- 22b: key groove portion
- 22c: key groove portion
- 22d: rear end portion
- 23: pressing button body
- 24: lock key
- 24a: engaging portion
- 24b: support point portion
- 24c: power point portion
- 25: lock key
- 25a: engaging portion
- 25b: support point portion
- 25c: power point portion
- 26a: projection
- 26b: projection
- 31: porous filter
- 31a: bottom surface
- 31b: plane
- 31c: plane
- 31d: one portion
- 31e: slit
- 32: blood collecting bottle
- 32a: side portion
- 32b: bottom portion
- 32c: leg portion

### DETAILED DESCRIPTION OF THE PREFERRED EMBODIMENTS

A blood collecting device and a blood collecting bottle of the present invention will be explained in detail with reference to the attached drawings hereinafter.

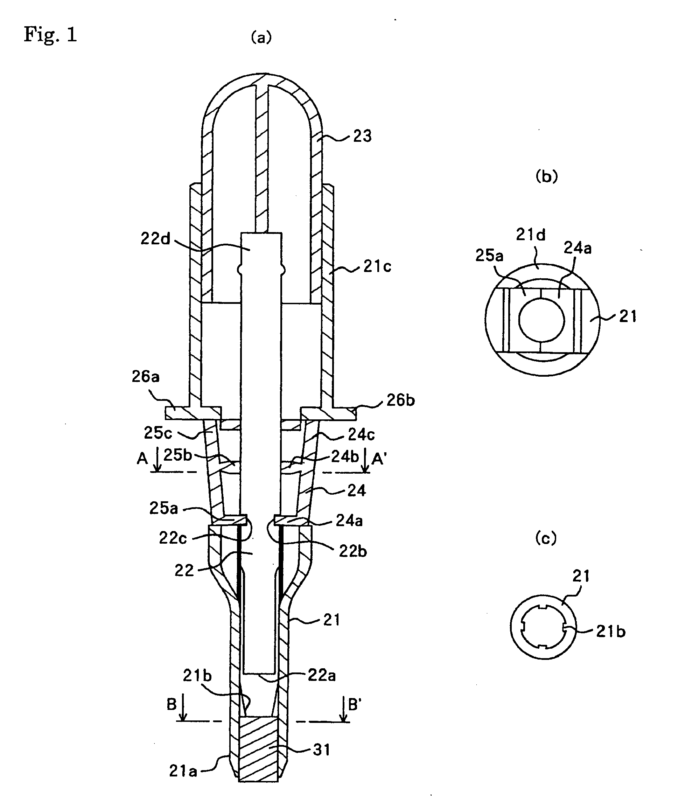

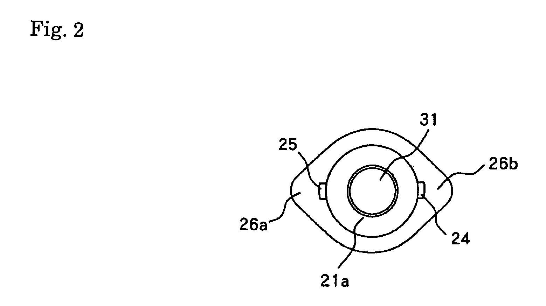

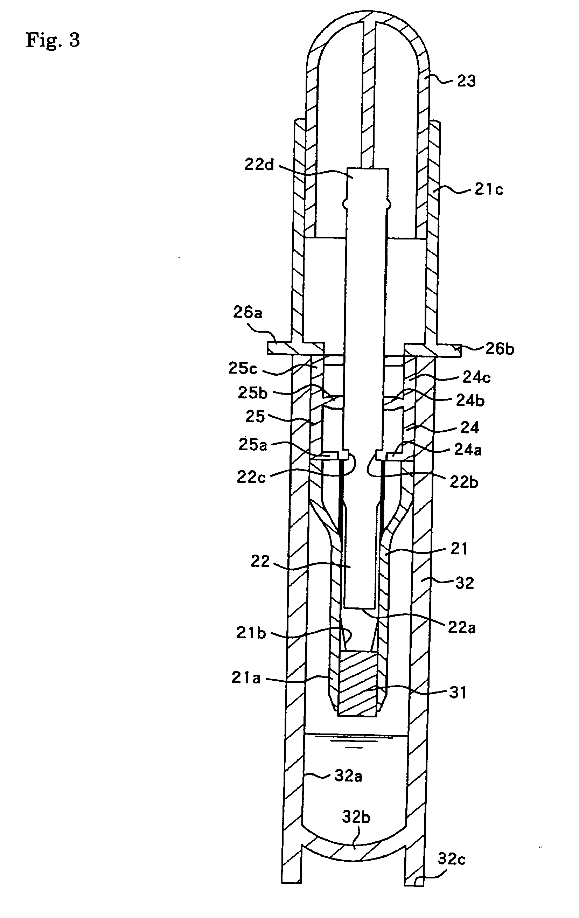

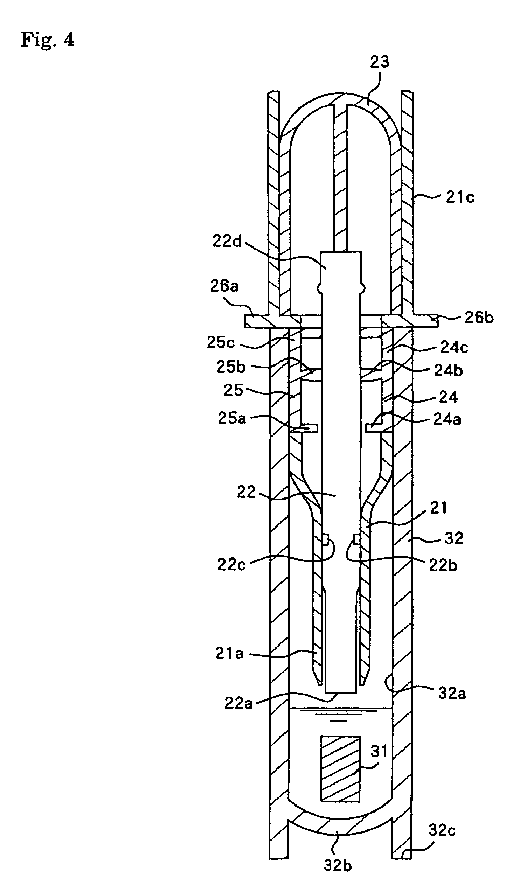

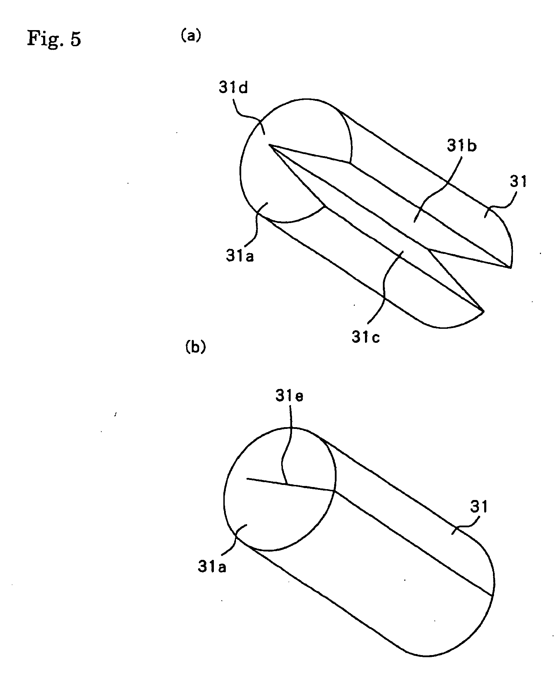

FIGS. 1A, 1B and 1C and 2 are schematic views of the blood collecting device of an embodiment according to the present invention. FIG. 1A is a sectional front view showing a state before blood collection and during the blood collection, and FIG. 2 is a bottom view. FIG. 5 is a perspective view of a liquid absorber (filter) which is used in the blood collecting device of the embodiment of the present invention. FIGS. 3 and 4 are schematic views of the blood collecting device and a blood collecting bottle of the embodiment according to the present invention. FIG. 3 is a sectional view showing a state in which the blood collecting device is inserted into the blood collecting bottle, and FIG. 4 is a sectional view showing a state in which a cap is pressed in and the liquid absorber is pushed out and separated after the blood collector is inserted into the blood collecting bottle.

An inner surface of a tip end portion 21a of a body 21 of the blood collecting device of the embodiment is in a columnar shape, and an outer surface is in a taper shape of which diameter becomes smaller toward the tip end portion. A columnar porous filter 31 is used as one embodiment of the liquid absorber. The porous filter 31 is inserted into an inside of the tip end portion 21a of the body 21, and one bottom surface 31a of the porous filter 31 is disposed to be located at an end of the tip end portion 21a. In the inside near the tip end portion 21a of the body 21, small projections 21b for determining the position in the direction of the center axis of the porous filter 31 are provided at four spots at intervals of 90° around the center axis (see FIG. 1C).

As the porous filter 31, a porous columnar body which is a commercially available product under the trade name of "Fiber Rod" or the like can be used, and the length is adjusted so that the porous filter 31 absorbs a predetermined amount of blood, for example, 1 µl, 2 µl, 5µl, 10 µl, 20 µl, 50 µl, 60 µl, 70 µl, 80 µl or 100 µl of blood by utilizing capillarity. Thereby, blood or the like in an amount with high accuracy can be easily collected with high repeatability. To prevent coagulation of blood, it is preferable to immerse the porous filter 31 in an anticoagulant such as heparin.

As a body fluid to be collected, pus, saliva, urine and the like can be cited other than blood, and the porous filter 31 should be used by changing the kind of the filter, the size of the holes, and the amount in accordance with the body fluid.

As shown in FIGS. 5A and 5B, the porous filter 31 is cut by planes 31b and 31c passing through the center axis with one portion 31d left to form a slit 31e. The slit can be changed to another form, for example, the slit is cut by three planes toward the center axis, two planes among them stay inside near the side surface and the other plane reaches the side surface.

A rear end portion 21c of the body is a cylindrical shape with a slightly large diameter, and a pressing button body 23 is inserted in its inner side.

The pressing button body 23 is made by connecting a cylindrical part and a semi-spherical shell part, and a rear end portion 22d of a pressing shaft portion 22 is inserted into and fixed to the cylindrical part. The pressing shaft portion 22 is disposed inside the body 21 movably in the center axis direction. A tip end of the pressing shaft portion 22 is a pressing portion 22a, and key groove portions 22b and 22c are symmetrically formed at a central part of the pressing shaft portion 22.

Lock keys 24 and 25 are symmetrically disposed at a central part of the body 21. The lock keys 24 and 25 are constructed by materials constituted of engaging portions 24a and 25a, support point portions 24b and 25b and power point portions 24c and 25c and having some flexibility, such as a resin or hard rubber.

FIG. 1B is a sectional view taken along the line A-A' in FIG. 1A. Tip ends of the support points 24b and 25b of the lock keys 24 and 25 abut on side surfaces of the pressing shaft portion 22. In the normal state, the engaging portions 24a and 25a of the lock keys 24 and 25 respectively engage in the key groove portions 22b and 22c of the pressing shaft portion 22. The lock key outer surfaces near the power point portions 24c and 25c are located at outer sides with respect to the center axis of the body, and the lock key outer surfaces near the engaging portions 24a and 25a are located at inner sides with respect to the center axis of the body. The outer surfaces of the lock keys 24 and 25 are located to be inclined with respect to the center axis of the body in the normal state. The engaging portions of the lock keys engage with the key groove portions of the pressing shaft portion in the normal state in this manner, and therefore, the pressing shaft portion cannot move in the direction of the center axis of the body.

At a rear end portion 21c of the body 21 of the blood collecting device of the embodiment, projections 26a and 26b which project outward are mounted to the positions in contact with the lock key 24 at an interval of 180° around the center axis. Therefore, even if the blood collector is placed on the desk, it is difficult to roll and fall from the edge of the desk.

The center of gravity of the blood collector of the embodiment is located near the projections 26a and 26b, or near to the rear end portion. Therefore, when the blood collector is placed on the desk, the tip end portion 21a and the porous filter 31 of the blood collector do not touch the surface of the desk, and are kept hygienic.

It is extremely easy to collect a predetermined amount of blood by using a blood collector of the embodiment according to the present invention. Skin is made to bleed by stinging it with a needle or the like, then the tip end portion 21a of the blood collecting device is brought into contact with the blood, and the blood is absorbed into the porous filter 31. When the porous filter is kept in contact with the blood, the porous filter 31 cannot absorb the blood any more. At that time, a predetermined amount of blood, 50 µl of blood is collected in the porous filter 31 with high accuracy. In addition, as the blood is absorbed from the one bottom surface 31a of the porous filter 31 by capillarity, air caught in holes in the porous filter 31 is driven toward the other bottom surface and gradually released from the other bottom surface. As a result, air is not included in the blood which is collected in the porous filter 31.

Next, as shown in FIG. 3, a blood collecting bottle 32 of the embodiment is in a cylindrical shape, and is constructed by a side portion 32a, a bottom portion 32b and a leg portion 32c. A predetermined drug solution is in the blood collecting bottle, and an opening is hermetically sealed by a sealing cap not shown.

The sealing cap of the blood collecting bottle 32 is detached and after the opening is opened, the blood collecting device of the embodiment is inserted into the blood collecting bottle 32. Then, the inner surface of the side portion 32a of the blood collecting bottle 32 contacts the power point portions 24c and 25c of the lock keys 24 and 25 of the blood collecting device, and as the blood collecting device is further inserted therein, the power point portions 24c and 25c are pressed and moved in the direction of the center axis of the blood collecting device. Then, the engaging portions 24a and 25a which are located at the opposite side from the power point portions 24c and 25c with the support point portions 24b and 25b therebetween move in the direction to be away from the center axis of the blood collecting device. As a result, engagement of the engaging portions 24a and 25a of the lock keys 24 and 25 and the key groove portions 22b and 22c of the pressing shaft portion 22 is released, and the pressing shaft portion 22 can freely and linearly move along the direction of the center axis of the blood collecting device. A linear guide groove may be provided at the inner surface of the body 21 so that the pressing shaft portion 22 does not rotate.

Subsequently, when the pressing button body 23 is pressed with respect to the body 21 with the lock means released, the pressing shaft portion 22 moves along the center axis of the body 21, the pressing portion 22a of the pressing shaft portion 22 abuts on the bottom surface behind the porous filter 31 to press the porous filter 31, and the porous filter 31 is pushed out and separated from the tip end portion 21a of the body 21.

Subsequently, the separated porous filter 31 drops into the drug solution which is housed in the blood collecting bottle. The slit 31e is formed at the porous filter 31, and therefore, in the drug solution, the porous filter 31 contacts the drug solution and opens along the slit 31e. Then, both the blood near the surface and the blood near the center of the blood collected in the porous filter 31 quickly elute into the drug solution. Accordingly, the collected blood can be evenly and uniformly treated with the drug solution.

The shape and structure of the leg portion 32c is designed so that the blood collecting bottle 32 can stably stand erect.

It is preferable that the tip end portion 21a of the body 21 of the blood collecting device is transparent or semitransparent so that the presence or absence of the porous filter 31 can be visually confirmed and the absorbing state of the blood or the like into the porous filter 31 is visually confirmed.

## Claims

1. A blood collecting device, wherein a columnar liquid absorber, which contains a blood coagulation preventing solution, has a slit, and temporarily absorbs a predetermined amount of blood without coagulation is disposed at a tip end, and a pressing body which presses a rear surface of the liquid absorber to separate the liquid absorber is provided.

2. The blood collecting device according to claim 1, wherein a position of the center of gravity is designed so that a tip end does not contact a surface of a table when the blood collecting device is placed on the table with its center axis nearly horizontal, and a projection which prevents the blood collecting device from rolling on the table is provided at a central part.

3. The blood collecting device according to claim 1 or 2, comprising: releasable lock means which stops movement of the pressing body to stop the pressing body from pressing the absorber, and releases the stoppage to make movement of the pressing body possible.

4. A blood collecting bottle, wherein when the blood collecting device according to claim 3 is pushed in and fitted in, the blood collecting device abuts on an inner wall and releasable lock means of the blood collecting device is released, and when the pressing body of the blood collecting device is pressed, a liquid absorber is separated from the tip end of the blood collecting device, and the liquid absorber is immersed in a contained drug solution, and the blood temporarily absorbed in the liquid absorber elutes into the drug solution.
